# NOUVEAU FASCICULE DE BREVET EUROPEEN

(11) **EP 0 299 816 B2**
(45) Date de publication et mention de la décision concernant l'opposition: **28.03.2001**
(45) Mention de la délivrance du brevet: 04.03.1992
(21) Numéro de dépôt: 88401503.3
(22) Date de dépôt: 16.06.1988
(51) Int. Cl.: A61K 7/155

(54) **Nouvelles compositions épilatoires**
Neue Haarentfernungsmittel
New depilatory compositions

(30) Priorité: 06.07.1987 FR 8709552
(43) Date de publication de la demande: 18.01.1989
(73) Titulaire: des Garets, Christian, F-75008 Paris (FR)
(72) Inventeur: des Garets, Christian, F-75008 Paris (FR)
(74) Mandataire: Armengaud Ainé, Alain

(56) Documents cités:
- CA-A- 1 185 186
- JP-A- 5 616 407
- US-A- 4 282 877
- CHEMICAL ABSTRACTS, vol. 104, 1986, page 377, résumé no. 74822g, Columbus, Ohio, US; & JP-A-60 202 810 (NICHIBAN CO., LTD) 14-10-1985
- CHEMICAL ABSTRACTS, vol. 94, 1981, page 366, résumé no. 180497q, Columbus, Ohio, US; & JP-A-81 16 407 (NITTO ELECTRIC INDUSTRIAL CO., LTD) 17-02-1981
- Article "Le collage industriel", Ed. Usine Nouvelle, pp. 85-93, Ph. Cognard et F. Pardos

## Description

La présente invention est relative à une nouvelle composition épilatoire, à son mode de préparation et à son mode d'emploi.

A l'heure actuelle, on utilise pour l'épilation des compositions généralement appelées "cires" qui sont à base de colophanes soit pures, soit d'abeille, cire minérale, huile, lanoline, etc...

Ces compositions sont appliquées à chaud, en couche relativement épaisse sur la peau et son réutilisables après filtration. L'épaisseur de la couche permet à la cire de ne pas refroidir trop vite et donc de garder une certaine température (25/30°C). Cette cire devient complètement dure et cassante et est donc inutilisable en l'état.

On utilise aussi d'autres compositions, généralement appelées cires tièdes, à base de colophane ou colophane modifiées, ou de résine synthétique, de produits gras, de miel et d'autres produits tels que glucose, miel, etc.

Ce type de cire s'applique en couche fine et se retire à l'aide d'une bande de matière cellulosique, notamment de "cellophane" ou de non-tissé, raison pour laquelle elle est appelée cire jetable.

US-A- 4282877 décrit un exemple de réalisation d'une telle cire sans bande comportant un adhésif constitué par de la colophane permettant d'arracher le poil qui adhère sur cet adhésif. Il est prévu d'ajouter à la composition un polymère de masse moléculaire élevée pour lui conférer une certaine cohésion.

CHEMICAL ABSTRACTS vol. 104, 1986, page 377, résumé n°74822 g, Columbus, Ohia, US décrit également une cire épilatoire s'appliquant sur la peau à l'aide d'une bande.

CA-A- 1185186 décrit une composition épilatoire qui s'applique sans bande sur la peau, cette composition comportant un agent collant, pour assurer l'arrachage du poil, qui est constitué par du sucre.

Le présent titulaire a par ailleurs proposé (voir le brevet européen numéro 86 400 311 publié sous le numéro 0 194 181) une nouvelle composition épilatoire dite "basse température" et "jetable", c'est à dire non destinée à être réutilisée après filtration, dont le constituant principal est un sel de colophane, et notamment un résinate de triéthanolamine, qui s'applique aussi en couche très fine et se retire à l'aide d'une bande de pellicule cellulosique notamment de "cellophane".

La présente invention se propose d'apporter une nouvelle génération de compositions à épiler, d'emploi beaucoup plus facile que les compositions épilatoires actuellement disponibles.

A cet effet, l'invention concerne une nouvelle composition épilatoire ou "cire à épiler" du type "jetable" pouvant être déposée en couche mince sur la surface de la peau, comportant un élastomère thermoplastique, de la colophane et des assouplissants et adoucissants, caractérisée par la composition suivante :
- Composé élastomère thermoplastique 5 à 15% en poids ;
- Colophane naturelle et/ou colophane modifiée 40 à 80% en poids.
- Assouplissant ou adoucissant choisi parmi graisses ou cire 10 à 20% en poids,
   le composé élastomère étant choisi dans les groupes comprenant :
- les copolymères d'éthylène-propylène ;
- les copolymères styrène-butadiène ;
- les copolymères éthylène-acétate de vinyle ;
- les caoutchoucs nitriles ;
- les élastomères thermoplastiques polyesters,
la composition résultante étant telle qu'après chauffage elle se présente sous forme d'un liquide onctueux qui peut être appliqué sur la peau sous forme de couche mince, qui après un temps de prise, a perdu son pouvoir collant dans sa surface extérieure en-dessus et en-dessous, et peut être alors détachée par pelage.

Ainsi l'invention concerne une nouvelle composition épilatoire ou "cire à épiler" du type "basse température" et "jetable" qui contient comme constituant principal un composé élastomère thermoplastique, permettant de contrôler l'élasticité de la composition à température ambiante et sans que cette cire se casse, grâce à l'homogénéité donnée au mélange ; auquel on ajoute à ce constituant principal
- de la colophane naturelle au modifiée donnant le pouvoir collant au produit ;
- des assouplissants et adoucissants tels que, par exemple, de la cire d'abeilles, ou de la cire micro-cristalline,
- éventuellement des charges (carbonate de calcium ou bioxyde de titane),
cette cire à épiler pouvant être déposée en couche mince, contrairement aux cires chaudes qui sont déposées en couche relativement épaisse ou aux cires tièdes dites jetables qui doivent être utilisées avec une bande de "cellophane".

La composition peut également comprendre des charges telles que notamment du carbone de calcium ou du bioxide de titane selon une proportion de l'ordre de 5 à 15%.

L'invention vise également un procédé d'épilation, caractérisée en ce qu'on chauffe une composition épilatoire telle que définie ci-dessus, pour qu'elle devienne un liquide onctueux, qu'on la dépose alors sur la partie à épiler à l'aide d'une spatule appropriée, ou d'un appareil distributeur, l'application étant réalisée en couche mince sur la peau en formant des bandes dont les dimensions varient selon la partie à épiler, qu'on laisse prendre la composition quelques instants, de manière à ce que la couche mince appliquée donne naissance par polymérisation à une mince pellicule plastique et qu'on détache, par pelage, cette mince pellicule qui a perdu son pouvoir collant dans sa surface extérieure en-dessus et en-dessous et qui emprisonne le poil sans adhérer à la peau, ce qui permet l'arrachage des poils emprisonnés dans la pellicule.

On peut aussi appliquer la cire à l'aide d'un appareil distributeur chauffant ou non et muni d'un réservoir pouvant contenir de la cire ; On laisse quelques instants la composition "prendre" de manière à ce que la couche mince donne naissance par polymérisation à une mince pellicule plastique qui emprisonne le poil sans adhérer à la peau, la bande ayant perdu son pouvoir collant, dans sa surface extérieure en dessus et en dessous. Il suffit ensuite de détacher par pelage la mince pellicule obtenue pour obtenir l'arrachage des poils emprisonnés dans la bande.

Il est possible, en remplaçant dans la formule mentionnée ci-dessus les colophanes par certains types de colophanes ou résines liquides, d'utiliser le produit à épiler selon l'invention à température ambiante, sans avoir besoin de le chauffer préalablement. Il faut toutefois que ce produit soit conservé à l'abri de l'air.

On a donné ci-après à titre d'exemple un mode de préparation d'une composition épilatoire selon la présente invention.

On commence tout d'abord par faire fondre l'élastomère choisi à une température comprise entre 90 et 160°C, en fonction de la nature dudit élastomère. On ajoute ensuite au produit ainsi obtenu l'ensemble des autres composants mentionnés ci-dessus, tout en maintenant la température du mélange à un minimum de plus ou moins 100°C. L'élastomère n'étant pas un produit soluble, il est nécessaire, afin de donner au produit épilatoire une grande homogénéité, de le mélanger à l'aide d'un agitateur approprié pendant un certain temps.

Il demeure bien entendu que l'invention n'est pas limitée aux exemples de réalisations décrits ci-dessus mais qu'elle en englobe toutes les variantes.

## Revendications

1. Composition épilatoire dite "cire à épiler", du type jetable pouvant être déposée en couche mince sur la surface de la peau, comportant un composé élastomère thermoplastique, de la colophane, et des assouplissants et adoucissants, caractérisée par la composition suivante:
- composé élastomère thermoplastique
5 à 15 % en poids
- Colophane naturelle et/ou colophane modifiée
40 à 80 % en poids
- Assouplissant ou adoucissant choisi parmi graisses ou cire
10 à 20 % en poids
le composé élastomère étant choisi dans les groupes comprenant
- les copolymères d'éthylène-propylène ;
- les copolymères styrène-butadiène ;
- les copolymères éthylène-acétate de vinyle ;
- les caoutchoucs nitriles ;
- les élastomères thermoplastiques polyesters,
la composition résultante étant telle qu'après chauffage elle se présente sous forme d'un liquide onctueux qui peut être appliqué sur la peau sous forme de couche mince, qui après un temps de prise, a perdu son pouvoir collant dans sa surface extérieure en-dessus et en-dessous, et peut être alors détachée par pelage.

2. Composition selon la revendication 1, caractérisée en ce que les assouplissants et adoucissants sont de la cire d'abeille, ou de la cire micro-cristalline.

3. Composition épilatoire selon la revendication 1 ou 2, caractérisée en ce qu'elle comprend en outre des charges, telles que notamment du carbonate de calcium ou du bioxyde de titane, selon une proportion de l'ordre de 5 à 15 % en poids.

4. Procédé d'épilation caractérisé en ce qu'on chauffe une composition épilatoire selon l'une quelconque des revendications 1 à 3, pour qu'elle devienne un liquide onctueux, qu'on la dépose alors sur la partie à épiler à l'aide d'une spatule appropriée, ou d'un appareil distributeur, l'application étant réalisée en couche mince sur la peau en formant des bandes dont les dimensions varient selon la partie à épiler, qu'on laisse prendre la composition quelques instants, de manière à ce que la couche mince appliquée donne naissance par polymérisation à une mince pellicule plastique et qu'on détache, par pelage, cette mince pellicule qui a perdu son pouvoir collant dans sa surface extérieure en-dessus et en-dessous et qui emprisonne le poil sans adhérer à la peau, ce qui permet l'arrachage des poils emprisonnés dans la pellicule.

## Patentansprüche

1. Haarentfernungsmittel, sogenanntes "Enthaarungswachs", vom Typ zum einmaligen Gebrauch, das als dünne Schicht auf die Oberfläche der Haut aufgebracht werden kann und eine thermoplastische Elastomerverbindung, Kolophonium und geschmeidig machende und weich machende Mittel enthält, gekennzeichnet durch die folgende Zusammensetzung:
- thermoplastische Elastomerverbindung 5 bis 15 Gewichtsprozent
- natürliches Kolophonium und/oder modifiziertes Kolophonium 40 bis 80 Gewichtsprozent
- geschmeidig machendes oder weich machendes Mittel, das ausgewählt ist aus Fetten oder Wachs 10 bis 20 Gewichtsprozent,
wobei die Elastomerverbindung ausgewählt ist aus den Gruppen, welche enthalten:
- die Ethylen-Propylen-Copolymeren
- die Styrol-Butadien-Copolymeren
- die Ethylen-Vinylacetat-Copolymeren
- die Nitrilkautschuke
- die thermoplastischen Polyester-Elastomeren,
wobei das erhaltene Mittel nach Erwärmen in Form einer salbenartigen Flüssigkeit vorliegt, die auf die Haut in Form einer dünnen Schicht aufgetragen werden kann, die nach einer Härtungszeit ihre Klebkraft auf ihrer oberen und unteren Außenfläche verloren hat und dann durch Abziehen entfernt werden kann.

2. Haarentfernungsmittel nach Anspruch 1, dadurch **gekennzeichnet**, daß die geschmeidig machenden und weich machenden Mittel Bienenwachs oder mikrokristallines Wachs sind.

3. Haarentfernungsmittel nach Anspruch 1 oder 2, dadurch **gekennzeichnet**, daß es außerdem Füllstoffe wie besonders Calciumcarbonat oder Titandioxid in einem Anteil in der Größenordnung von 5 bis 15 Gewichtsprozent enthält.

4. Verfahren zum Entfernen von Haaren, dadurch **gekennzeichnet**, daß man ein Haarentfernungsmittel nach einem der Ansprüche 1 bis 3 erwärmt, so daß es zu einer salbenartigen Flüssigkeit wird, die man dann auf den zu enthaarenden Teil mittels eines geeigneten Spatels oder Verteilungsgeräts aufbringt, wobei die Anwendung in einer dünnen Schicht auf der Haut unter Ausbildung von Streifen realisiert wird, deren Abmessungen entsprechend dem zu enthaarenden Teil verschieden sind, daß man das Mittel einige Augenblicke härten läßt, so daß die dünne aufgebrachte Schicht durch Polymerisation eine dünne Kunststoffhaut liefert und daß man durch Abziehen diese dünne Haut entfernt, die auf ihre Außenfläche oben und unten ihre Klebkraft verloren hat und welche die Haare festhält ohne an der Haut zu haften, was das Herausreißen der in der dünnen Haut festgehaltenen Haare ermöglicht.

## Claims

1. A disposable depilatory composition, called "wax removing hair", which can be applied as a thin layer on the surface of the skin, comprising a thermoplastic elastomeric compound, rosin and softeners and sweeteners, characterized by the following composition : which is removable by peeling away from the surface of the skin after setting to a thin plastic layer and losing its tackiness, said composition conciciting essentially of :
- thermoplastic elastomeric compound
5 to 15% by weight ;
- natural rosin and/or modified rosin
40 to 80% by weight ;
- softener or sweetener selected from fats or wax
10 to 20% by weight,
said elastomeric compound being selected from the groups comprising :
- ethylene-propylene copolymers ;
- styrene-butadiene copolymers ;
- vinyl ethylene-acetate copolymers ;
- nitrile rubbers ;
- polyester thermoplastic elastomers ;
the resulting composition being such that, after heating, it is under the form of an unctuous liquid which can be applied on the skin under the form of a thin layer, which after a setting time, has lost its tackiness in its outer surface on top and underneath, and can thus be removed by peeling.

2. The composition of claim 1, wherein said softeners and sweeteners are bees-wax or microcrystalline wax.

3. Depilatory composition according to claim 1 or 2, further comprising fillers, particularly such as calcium carbonate or titane dioxide, in a proportion of about 5 to 15% by weight.

4. A process for removing hair comprising heating a depilatory composition according to anyone of claims 1 to 3, so that it becomes an unctuous liquid, then applying the same on the part to be depilated with an appropiate spatula or a distributing apparatus, said application being carried out as a thin film on the skin, by forming strips whose dimensions vary depending on the part to be depilated, leaving the composition to set for a few moments, so that the thin film applied gives rise through polymerization to a thin plastic layer and removing, by peeling, said thin layer which has lost its tackiness in its outer surface on top and underneath and which catches the hair without adhering to the skin, thus enabling the hair captured in the film to be removed.
